# EUROPEAN PATENT SPECIFICATION

(11) **EP 0 437 334 B1**
(45) Date of publication and mention of the grant of the patent: **23.08.1995**
(21) Application number: 91300093.1
(22) Date of filing: 07.01.1991
(51) Int. Cl.: C11C 1/00, C11C 1/08, C11B 1/10, A61K 31/23, C07C 51/48, C07C 53/126, A23L 1/03

(54) **Method of producing a fatty acid**
Verfahren zur Herstellung einer Fettsäure
Procédé de préparation d'un acide gras

(30) Priority: 06.01.1990 GB 9000309
(43) Date of publication of application: 17.07.1991
(73) Proprietor: THE SCOTTISH AGRICULTURAL COLLEGE, Edinburgh EH9 3JG, Scotland (GB)
(72) Inventor: Noble, Raymond Clifford, Ayr Scotland (GB); Cocchi, Massimo, Bologna (IT)
(74) Representative: Pattullo, Norman

(56) References cited:
- EP-A- 0 342 795
- WO-A-89/05655
- FR-A- 2 599 382
- WORLD PATENTS INDEX LATEST, Section Ch, Week 8933, 1989, Derwent Publications Ltd., London, GB; Class D, AN 89-307256& SE-A-8 705 122 (NUTRITIONAL RES. INST.) 23-06-1989
- JOURNAL OF BIOLOGICAL CHEMISTRY, vol. 226, no. 1, 1957, BALTIMORE, US, pages 497-509; J. FOLCH et al.: "A simple method for the isolation and purification of total lipids from animal tissues"

## Description

This invention relates to a method of producing a fatty acid in treating the body.

Fatty acids play an important role in the functioning of human and animal bodies, and the particular fatty acid with which this invention is concerned, namely docosahexaenoic acid (DHA), is known to have beneficial effect on at least the retina, gonads and brain. It is also known that the levels of this fatty acid reduce with age, and it is therefore desirable to supplement these levels by introduction from other sources.

Fish oil, for example, contains significant amounts of DHA but does not contain a compatible solubilising agent which is necessary for the incorporation of the DHA into the tissues of the body; DHA from this source is not therefore a practical proposition.

It has been suggested to extract DHA attached to phospholipid from young cow brains, and in this combination the DHA is solubilised and is therefore easily received into the body. In addition it has been found that the combined DHA-phospholipid finds its way naturally to the target organs which require enhancement of DHA; in the absence of the phospholipid this does not happen and DHA solubilised with other materials such as emulsifiers is not taken up by the target organs. However, only very small amounts of DHA are obtainable from cow brains, and the economic value of cows in their other functions outweighs the benefit to be derived by killing them early in life to obtain the DHA.

According to the present invention there is provided a method of producing DHA for use in introduction into human or animal bodies in a solubilised form, comprising extracting the DHA attached to phospholipid from brains of pre-adult hatched chickens. The combined DHA-phospholipid can then be introduced for example orally, intravenously or parenterally into a body such as the retina, the gonads and the brain. The presence of phospholipid is itself beneficial as well as acting to guide the DHA to the desired areas.

Further according to the present invention there is provided the use of combined DHA-phospholipid obtained by the method above for the manufacture of a medicament for the treatment of a human or animal body.

The levels of DHA in young chicken brains are remarkable and surprisingly high, being of the order of up to 26% of the total fatty acid content, and its recovery is accordingly a practical commercial possibility. The commercial feasibility is enhanced by the fact that the majority of chicks are hatched in controlled environments producing many thousands of chicks daily, and approximately one-half of these, the male chicks, are currently of no commercial use and are killed early in life for no return. The present invention therefore provides a substantial means of obtaining an economic benefit from these male chicks.

As the chick embryos hatch and grow to maturity the level of DHA in their brains diminishes, and the most effective time to extract the DHA is shortly after hatching; this again adds to the economic benefits of the invention as it is not necessary to maintain the life of male chicks beyond that at present.

The DHA attached to phospholipid from pre-adult chicken brains and each single phospholipid fraction of the same source is conveniently in a form suitable for oral, intravenous or parental administration in a suitable pharmaceutical or dietetic vehicle, for example tablets, capsules, powder preparation, and lyophilized preparations to be diluted in appropriate solvents or in phials.

Advantageously a preservative may be incorporated in the preparation. Antioxidant (natural or synthetic) agents have been found suitable for the purpose.

Preferably the DHA is extracted from chicks up to three days after hatching, preferably up to two days after hatching.

An embodiment of the present invention will now be described by way of illustration in the following Example.

### EXAMPLE

1. Sample: complete brain from day old male chicks.
2. Total lipid extraction: the whole brain tissue was homogenised and refluxed in 2:1 (v/v) chloroform:methanol, the non-lipid contaminants were removed by filtration and subsequent washing of the eluant with 0.88 per cent (w/v) potassium chloride and a lipid-rich chloroform phase was obtained by centrifugation at low speed.
3. Phospholipid preparation and analysis: the total lipids were fractionated into the major lipid classes on thin layer chromatoplates of silica gel G, thickness 0.25 mm, using a solvent system of hexane: diethyl ether: formic acid (80:20: 1 v/v/v). Following visualisation under ultraviolet light after spraying with 0.1 per cent w/v solution of 2,7-dichlorofluorescein in methanol, the phospholipid was eluted from the silica gel by washing with 3 x 5 ml of chloroform: methanol: water (5:5:1 v/v/v). The phospholipid was transmethylated by refluxing with methanol: toluene: sulphuric acid (20:10:1 v/v/v). Gas liquid chromatography of the methyl fatty acid ester derivatives on a packed column of 15 per cent CP Sil 84 on Chromosorb WHP enabled the quantification of the relative proportions of the major long chain fatty acids present.
4. Fatty acid composition: the proportion (per cent) by weight of the major fatty acids was - palmitic 24.3, palmitoleic acid 2.3, stearic acid 17.8, oleic acid 14.7, linoleic acid 1.4, linolenic acid 0.5, arachidonic acid 11.1, docosahexaenoic acid 23.1.

The DHA-phospholipid obtained by this method was then tested as follows.

Sixteen rats, all 18 months old, were divided into two groups of eight. To one group the DHA-phospholipid sonicated in a physiological medium was administered by intraperitoneal injection at a dosage of 50 mg DHA-phospholipid each day for 7 days; 25% by weight of the DHA-phospholipid consisted of DHA.

The other group of rats received injections of the same amount of physiological medium as the test group, but without the DHA-phospholipid.

After one week the rats' blood was sampled by heart puncture and the rats were killed by decapitation. Tissue was taken for analysis and plasma was obtained by centrifugation of the blood.

Results showed that triglyceride levels were 115 mg per 100 ml in the test group and 315 mg per 100 ml in the control group, representing a reduction of 63% in the test group. A marked reduction was also observed in the Holman Index (triene/tetraene fatty acid ratio) in the test group.

The fatty acid profile of the brain, gonads and retina showed significant improvement in the DHA content in the test group as compared to the controls.

Diseases and disorders for which the DHA-phospholipid obtained in the present invention is effective are, for example:
1. Degenerative diseases of retina caused by ageing;
2. Degenerative diseases of brain and nervous system caused by ageing;
3. Degenerative diseases of gonads caused by ageing and male infertility;
4. Nutrition care for the critically ill;
5. Organ failure induced by metabolic stress of cell membrane;
6. Acute inflammatory response;
7. Viral infection;
8. Diabetic retinopathy;
9. Hypertriglyceridemia;
10. Other conditions which reduce the DHA synthesis from its precursor by the blockage of Delta 6, Delta 5, Delta 4, Desaturase enzyme.

## Claims

1. A method of producing docosahexanoic acid for use in introduction into human or animal bodies in a solubilised form, comprising extracting the docosahexanoic acid attached to phospholipid from brains of pre-adult hatched chickens.

2. A method according to Claim 1, wherein after extraction the docosahexanoic acid attached to the phospholipid is made into a form suitable for oral, intravenous or parenteral administration.

3. A method according to Claim 2, wherein the docosahexanoic acid attached to the phospholipid is made into the form of tablets, capsules, powder or solution.

4. A method according to Claim 2 or 3, wherein a preservative is included in said form suitable for oral or parenteral administration.

5. A method according to any one of the preceding Claims, wherein the brains are male chicken brains.

6. A method according to any one of the preceding Claims, wherein the extraction is performed by homogenising the brain tissue, removing non-lipid components, fractionating into major lipid classes and eluting the DHA-phospholipid.

7. A method according to any one of the preceding Claims, wherein the extraction is performed using chicks up to 3 days after hatching.

8. A method according to Claim 7, wherein the extraction is performed using chicks within 2 days of hatching.

9. The use of combined DHA-phospholipid obtained by the method of any one of Claims 1 to 8 for the manufacture of a medicament for the treatment of a human or animal body.

## Patentansprüche

1. Verfahren zum Herstellen von Docosahexanonsäure zur Verwendung bei der Einführung in menschliche und tierische Körper in einer löslich gemachten Form, welches aus einem Extrahieren der an Phospholipid gebundenen Docosahexanonsäure von den Gehirnen von nicht erwachsenen Brutküken besteht.

2. Verfahren nach Anspruch 1, bei welchem nach dem Extrahieren die an das Phospholipid gebundene Docosahexanonsäure in eine Form gebracht wird, die für eine orale, intravenöse oder parenterale Verabreichung geeignet ist.

3. Verfahren nach Anspruch 2, bei welchem die an das Phospholipid gebundene Docosahexanonsäure in die Form von Tabletten, Kapseln, Pulver oder eine Lösung gebracht wird.

4. Verfahren nach Anspruch 2 oder 3, bei welchem ein Konservierungsmittel in die für eine orale oder parenterale Verabreichung geeignete Form eingeschlossen ist.

5. Verfahren nach einem der vorhergehenden Ansprüche, bei welchem die Gehirne männliche Kükengehirne sind.

6. Verfahren nach einem der vorhergehenden Ansprüche, bei welchem das Extrahieren durch ein Homogenisieren des Gehirngewebes durchgeführt wird sowie ein Entfernen von nichtlipiden Komponenten, ein Fraktionieren in Hauptlipidklassen und ein Eluieren des DHA-Phospholipids.

7. Verfahren nach einem der vorhergehenden Ansprüche, bei welchem das Extrahieren unter Verwendung von Küken bis zu 3 Tage nach dem Ausbrüten durchgeführt wird.

8. Verfahren nach Anspruch 7, bei welchem das Extrahieren unter Verwendung von Küken innerhalb von zwei Tagen nach dem Ausbrüten durchgeführt wird.

9. Verwendung des kombinierten DHA-Phospholipids, erhalten durch das Verfahren nach einem der Ansprüche 1 bis 8, für die Herstellung eines Arzneimittels für die Behandlung eines menschlichen oder tierischen Körpers.

## Revendications

1. Procédé de préparation d'acide docosahexanoïque destiné à être utilisé pour l'introduction dans le corps humain ou animal sous une forme solubilisée, comprenant l'extraction d'acide docosahexanoïque attaché aux phospholipides à partir de cervelles de poussins couvés pas encore adultes.

2. Procédé selon la revendication 1, dans lequel, après extraction, l'acide docosahexanoïque attaché aux phospholipides est amené sous une forme appropriée pour une administration orale, intraveineuse ou parentérale.

3. Procédé selon la revendication 2, dans lequel l'acide docosahexanoïque attaché aux phospholipides est amené sous la forme de tablettes, capsules, poudre ou solution.

4. Procédé selon la revendication 2 ou la revendication 3, dans lequel on inclut un agent préservatif dans ladite forme appropriée pour une administration orale ou parentérale.

5. Procédé selon l'une quelconque des revendications précédentes, dans lequel les cervelles sont des cervelles de poussins mâles.

6. Procédé selon l'une quelconque des revendications précédentes, dans lequel l'extraction est réalisée en homogénéisant les tissus de cervelles, en enlevant les composants non lipides, en fractionnant selon les classes principales de lipides et en éluant le phospholipide-DHA.

7. Procédé selon l'une quelconque des revendications précédentes, dans lequel l'extraction est réalisée en utilisant des poussins jusqu'à trois jours après la couvée.

8. Procédé selon la revendication 7, dans lequel l'extraction est réalisée en utilisant des poussins au plus tard deux jours après la couvée.

9. Utilisation du combiné DHA-phospholipide obtenu par le procédé selon l'une quelconque des revendications 1 à 8 pour la fabrication d'un médicament pour le traitement du corps humain ou animal.
